# EUROPÄISCHE PATENTSCHRIFT

(11) **EP 2 014 758 B1**
(45) Veröffentlichungstag und Bekanntmachung des Hinweises auf die Patenterteilung: **24.11.2010**
(21) Anmeldenummer: 08012546.1
(22) Anmeldetag: 11.07.2008
(51) Int. Cl.: C11D 17/00, C11D 17/04, A61L 9/05

(54) **Stückförmiges Reinigungsmittel für den Sanitärbereich mit einem Werbeträger**
Cleaning block for sanitary use with an advertising medium
Produit de nettoyage en bloc pour le domaine sanitaire doté d'un support publicitaire

(30) Priorität: 13.07.2007 DE 102007033077
(43) Veröffentlichungstag der Anmeldung: 14.01.2009
(73) Patentinhaber: Buck-Chemie GmbH, 71083 Herrenberg (DE)
(72) Erfinder: Jaeschke, Edgar, 70794 Filderstadt (DE); Leipold, Joachim, 72760 Reutlingen (DE); Fritz, Matthias, 72810 Gomaringen (DE); Kauth, Simon, 72116 Mössingen (DE)
(74) Vertreter: Mammel, Ulrike

(56) Entgegenhaltungen:
- WO-A-97/20029
- WO-A-02/061030
- DE-A1-102004 035 655
- DE-C- 265 428
- US-A- 5 869 437

## Beschreibung

Die vorliegende Erfindung betrifft ein stückförmiges Mittel zum Reinigen und/oder Beduften für den Sanitärbereich mit einem Werbeträger.

Im Stand der Technik sind eine Vielzahl von Mitteln bekannt, die zur Reinigung und Beduftung von Toiletten dienen und die üblicherweise in käfigartigen Behältnissen, so genannten WC-Körbchen, am Rand der Toilette eingehängt werden (Rimblocks). Diese Mittel sind häufig einphasige Mittel; beim Überspülen mit Wasser werden sowohl die reinigenden Wirkstoffe, als auch die Duftstoffe nach und nach freigesetzt.

Rimblocks verbrauchen sich im Allgemeinen nach ca. 250 Spülungen, d.h. in einem Vier-Personen-Haushalt in der Regel in 4 bis 6 Wochen.

Im Handel werden Rimblocks sowohl zusammen mit den dazu passenden Körbchen, als auch als solche in Nachfüllpacks angeboten. Nachdem jedoch von den unterschiedlichen Herstellern WC-Körbchen in den verschiedensten Ausmaßen angeboten werden, steht der Verbraucher beim Besorgen der Nachfüllpackung nicht selten vor dem Problem, dass er sich nicht mehr erinnern kann, welches Nachfüllprodukt nun in sein WC-Körbchen passt.

Um dieses Problem zu lösen und auch die jeweiligen Toilettenreinigungsmittel zu individualisieren gibt es stückförmige Toilettenreinigungsmittel, die als Werbeträger z.B. eine Prägung an der Außenseite aufweisen. Nachteilig an der Prägung ist jedoch, dass diese beim Überspülen mit Wasser relativ schnell abgespült wird, so dass für den Verbraucher gerade gegen Ende der Benutzungsdauer nicht mehr erkennbar ist, um was für ein Mittel es sich gehandelt hat.

Weiterhin gibt es stückförmige Reinigungsmittel, die einen Werbeträger aus Plastik, der auf der Oberfläche oder im Innern des Mittels eingebracht sein kann, umfassen oder einen Werbeträger in Form eines auf der Oberfläche aufgeklebten Papieretiketts. Bei diesen Mitteln wird das Reinigungsmittel nach und nach abgespült, und der Werbeträger oder das Papieretikett bleiben im Körbchen zurück. Hierdurch wird zwar am Ende der Benutzung durch den verbleibenden Werbeträger eine Bestimmung der Herkunft ermöglicht. Allerdings muss der Werbeträger am Ende der Benutzung aus dem Körbchen entnommen und anschließend entsorgt werden, was vom Verbraucher als unhygienisch empfunden wird.

Aus der US 5,869,437 ist eine transparente Seife bekannt, in die ein Werbeträger aus einem wasserlöslichen Polymermaterial, nämlich ein sehr dünnes bedrucktes Blatt aus Polyvinylalkohol, eingebettet ist.

Die DE 265428 lehrt ein durchsichtiges Seifenstück mit einem darin eingebetteten, äußerst dünnen, flachen, durchsichtigen Etikett aus Gelatine.

Die Aufgabe der vorliegenden Erfindung besteht darin, ein optisch ansprechendes stückförmiges Toilettenreinigungsmittel mit einem Werbeträger bereitzustellen, das dem Verbraucher auf hygienische Weise während des Gebrauchs ein Wiedererkennen ermöglicht.

Diese Aufgabe wird durch ein stückförmiges Reinigungsmittel für den Sanitärbereich gelöst, das wenigstens eine im Wesentlichen transparente Phase aufweist und wenigstens einen Werbeträger, wobei die transparente Phase Reinigungsmittel umfasst und wasserlöslich oder wasserdispergierbar ist und der Werbeträger ein durch thermische Verformung hergestelltes Formteil ist, welches wenigstens ein wasserlösliches oder wasserdispergierbares Polymermaterial aus der Gruppe der Lignin oder Lignocellulose enthaltenden Polymere, der wasserlöslichen Polyurethane, der Polyethylenoxide oder der Polyetheroxide enthält, wobei der Werbeträger von der transparenten Phase wenigstens teilweise umschlossen ist, der Werbeträger wenigstens bereichsweise durch die transparente Phase von außen sichtbar ist und der Werbeträger in das Mittel eingebettet ist.

Durch die Verwendung eines Werbeträgers, der in das Mittel eingebettet ist und von der transparenten Phase wenigstens teilweise umschlossen ist, ist der Werbeträger von außen sichtbar, so dass sich der Verbraucher den Werbeträger über die Gebrauchsdauer des Mittels einprägen kann.

Die Masse, aus der der Werbeträger hergestellt ist, umfasst ein wasserlösliches oder wasserdispergierbares Polymermaterial, damit er sich nach und nach auflöst oder zerfällt und aus dem Körbchen herausgespült wird und nicht als zu entsorgender Rest vom Verbraucher aus dem Toilettenkörbchen entnommen werden muss.

Selbstverständlich müssen die Auflösegeschwindigkeiten des Werbeträgers, des Mittels und der transparenten Phase aufeinander abgestimmt werden. Ist der Werbeträger vollständig in das Mittel eingebettet, das heißt, von dem Mittel vollständig umgeben, so wird er erst dann freigespült, wenn das Mittel oder die transparente Phase an einer Seite des Werbeträgers aufgelöst ist. Wenn die Auflösung dieser an den Werbeträger angrenzenden, als erstes aufzulösenden Seite erst gegen Ende der Lebensdauer des Mittels erfolgt, das heißt, der Werbeträger über den größten Teil der Lebensdauer des Mittels durch das umgebende Mittel gegen Wasser abgeschirmt ist, so sollte sich der Werbeträger selbst relativ gut (und schnell) auflösen, da das Mittel beim Freispülen des Werbeträgers dann ja bereits fast vollständig verbraucht ist.

Ist der Werbeträger hingegen nicht vollständig in dem Mittel eingebettet und somit bereits beim ersten Gebrauch des Mittels in direktem Kontakt mit Wasser, so ist die Auflösegeschwindigkeit des Werbeträgers wenigstens bereichsweise herabzusetzen, so dass Reinigungsmittel und Werbeträger ähnliche Lebensdauern aufweisen.

Unter "teilweise umschlossen" wird verstanden, dass der Werbeträger wenigstens bereichsweise an die transparente Phase angrenzt, d.h. dass der Werbeträger und die transparente Phase wenigstens eine Berührungsfläche aufweisen.

Dabei ist die transparente Masse in Blickrichtung des Konsumenten vorzugsweise vor dem Werbeträger angeordnet.

Soll die dreidimensionale Form des Werbeträgers von außen sichtbar sein, so weisen Werbeträger und transparente Phase vorzugsweise mehrere Berührungsflächen auf oder in alle drei Raumrichtungen gekrümmte Berührungsflächen.

Unter "eingebettet" wird verstanden, dass der Formkörper vollständig oder wenigstens bezüglich seiner Oberfläche zu mehr als 60 %, vorzugsweise zu mehr als 80 % und besonders bevorzugt zu wenigstens 90 % von dem Mittel umgeben ist.

Der durch thermische Verformung erhältliche Formkörper kann beispielsweise durch thermisches Pressen, Blasverfahren, Spritzguss oder Tiefziehen hergestellt werden, wobei Spritzguss wegen der einfachen und kostengünstigen Herstellung bevorzugt ist.

Der Werbeträger sollte sich von der transparenten Phase und dem Untergrund abheben, so dass er vorzugsweise farbig ist oder sich farblich vom Hintergrund unterscheidet.

Dadurch, dass der Werbeträger in dem Körbchen für den Verbraucher bis zu Auflösung des Mittels sichtbar ist und sich der Verbraucher auch an diesen Werbeträger gewöhnt, treten die beschriebenen Schwierigkeiten bei der Auswahl der passenden Nachfüllpackungen nicht mehr auf.

Um das Wiedererkennen zu ermöglichen sollte der Verbraucher von der Sichtseite des Mittels wenigstens einen Teil des Werbeträgers, vorzugsweise jedoch den gesamten Werbeträger, erkennen können.

Hierzu weist das Mittel wenigstens bereichsweise auf einer Seite des Mittels, insbesondere der Sichtseite, eine transparente Phase auf. Vorzugsweise bedeckt die transparente Phase den Werbeträger vollständig auf wenigstens einer Seite. Die transparente Phase kann, muss aber nicht, eine Reinigungsmittel enthaltende Phase sein.

In einer weiteren Variante ist der Werbekörper mit mindestens zwei, vorzugsweise drei Seiten von einer oder mehreren transparenten Phasen kontaktiert. Auf der/den anderen Seite(n) des Werbeträgers kann beispielsweise eine nichttransparente Reinigungsmittelformkörperphase vorgesehen sein, beispielsweise auf der Basis der herkömmlichen Rezepturen von extrudierten Rimblocks. Damit kann das Mittel in dieser Variante aus einer nichttransparenten Reinigungsmittelformkörperschicht bestehen, auf deren einer Seite der Werbeträger wenigstens teilflächig aufliegen, wobei diese Seite der Reinigungsmittelformkörperschicht mit dem Werbeträger mit einer transparenten Phase beschichtet oder vergossen ist.

In einer weiteren Variante weist das Mittel nur eine oder mehrere transparente Phasen auf, in die der Werbeträger eingebettet ist und keine zusätzliche nichttransparente Reinigungsmittelformkörperphase. Diese Variante ermöglicht somit von allen Seiten des Mittels Sicht auf den Werbeträger.

Selbstverständlich kann das erfindungsgemäße Mittel auch mehrere Werbeträger umfassen.

Vorzugsweise ist die Auflösegeschwindigkeit des Mittels im Wesentlichen linear, das heißt, dass pro Spülzyklus prozentual immer in etwa derselbe Anteil des Mittels abgespült wird.

Die transparente Phase dient hauptsächlich dazu, den/die wasserlöslichen oder wasserdispergierbaren Werbeträger zunächst gegen das Spülwasser zu schützen und fortwährend Sicht auf den farbigen Werbeträger zu ermöglichen.

Darüber hinaus bewirkt die transparente Phase häufig auch eine Fixierung des Werbeträgers.

Eine Phase ist in Bezug auf die erforderliche Schichtdicke der Phase dann hinreichend transparent, wenn der Werbeträger durch die transparente Phase von außen erkennbar ist.

Die transparente Phase ist ebenfalls wasserlöslich oder -dispergierbar, damit sich diese ebenfalls nach und nach auflöst und gegen Ende des eigenen Auflösevorgangs dann den wasserlöslichen Werbeträger zur Auflösung durch das Spülwasser freigibt.

Die Masse des Werbeträgers kann zwischen 1 und 90 Gew.%, vorzugsweise zwischen 5 und 80 Gew.% und besonders bevorzugt zwischen 10 und 60 Gew.% der Gesamtmasse des Mittels betragen.

Der Anteil an transparenter Phase oder transparenten Phasen kann bis zu 95 Gew.% betragen, insbesondere, wenn außer dem Werbeträger keine weitere nichttransparente Phase in dem Mittel enthalten ist. Andererseits kann der Anteil der transparenten Phase auch gering sein und beispielsweise zwischen 0,5 Gew.% und 5 Gew.% liegen, insbesondere, wenn die transparente Phase den Werbeträger nur als dünne Schicht umhüllt.

Soweit das Mittel neben dem Werbeträger und der transparenten Phase noch eine weitere nichttransparente Phasen umfasst, betragen die Gew.% -Anteile des Werbeträgers zwischen 2 und 90 Gew.%, vorzugsweise zwischen 5 und 80 Gew.%, der transparenten Phase zwischen 0,2 und 90 Gew.%, vorzugsweise zwischen 5 und 80 Gew.% und der oder den nichttransparenten Phasen zwischen 0,5 und 90 Gew.%, vorzugsweise zwischen 5 und 80 Gew.%.

Der Werbeträger als ganzes oder Teile davon sind vorzugsweise spritzgegossen und teilweise aus einem wasserlöslichen oder wasserdispergierbaren Polymermaterial aufgebaut, so dass er sich in Kontakt mit Wasser vollständig auflösen oder dispergieren kann und dabei ggf. einen weiteren Wirkstoff freisetzen kann.

Selbstverständlich kann das erfindungsgemäße Mittel neben der einen transparenten Phase und dem einen Werbeträger auch weitere Phasen oder Schichten oder Werbeträger aufweisen, wobei sich diese auch wiederholen können. Die unterschiedlichen Schichten/Phasen/Werbeträger können sich auch durch eine unterschiedliche Beduftung auszeichnen, wodurch auch ein geruchlicher End-of-life-Indikator bereitgestellt werden kann.

Der Spritzgusskörper kann verschiedenartig geformt sein, beispielsweise als quader-, tabletten-, würfel- oder zylindrisch kompakter Körper. Auch kann das Mittel in Form einer zweidimensionalen Figur wie einer Tierfigur, einer Blume etc. ausgebildet sein, die sich über eine konstante Schichtdicke in die dritte Dimension erstreckt.

Weiterhin ist möglich, den Körper auch in der dritten Dimension variabel zu gestalten und somit einen in alle drei Raumrichtungen unterschiedlich geformten kompakten 3D-Körper herzustellen. Diese Variante eignet sich insbesondere für die Einbettung in einem vollständig transparenten Mittel. Der 3D-Körper kann auch hohl sein und mit einem oder mehren Wirkstoffen befüllt sein.

Durch das Spritzgießen steht eine ungeheure Formenvielfalt für das Mittel zur Verfügung, so dass der Werbeträger beispielsweise auch in Form des Firmenlogos, einer Marke, eines Schriftzuges oder sonstiger individualisierender oder optisch ansprechender Symbole hergestellt werden kann.

Die Außenmaße des 3D-Körpers können in weiten Grenzen variieren, beispielsweise zwischen 10 und 50 mm (Höhe) und 10 und 150 mm (Breite). Die Schichtdicke des Werbeträgers liegt zwischen 3 und 50 mm.

Sofern durch thermisches Verformen Platten- oder Folienmaterialien erhalten werden, können diese auch mit spanenden Verfahren oder über Stanzen weiter bearbeitet werden. Auch ist es möglich, die Plattenmaterialien thermisch weiter umzuformen, um z.B. gebogenen 3D-Formen zu erhalten.

Auch kann der aus wasserlöslichen oder wasserdispergierbaren Polymeren bestehende Werbeträger durch Stanzen, Fräsen oder ähnliche Verfahren aus entsprechend gegossenen oder extrudierten Platten oder Körpern hergestellt werden.

Weiterhin können die Formteile mit Haltevorrichtungen wie Widerhaken, Borsten etc. ausgerüstet sein, um zusätzliche mechanische Anker zu erhalten.

Über die Form des Werbeträgers besteht auch die Möglichkeit, Produkteigenschaften darzustellen, beispielsweise Tannenzapfenform für Tannenbaumdüfte wie beispielsweise Koniferen, Kiefer, Wacholder nachzubilden.

Die Auflöse- oder Dispergiergeschwindigkeit des Werbeträgers hängt u.a. von der Löslichkeit und Lösungstemperatur des wasserlöslichen Polymermaterials, den weiteren Bestandteilen und der Größe und Oberfläche des Werbeträgers ab.

Vorzugsweise liegen die Spülzahlen der reinen Werbeträger zwischen 70 und 200.

Als Polymermaterialien kommen Materialen in Frage, die sich unter den gegebenen Bedingungen eines Reinigungsvorgangs in wässriger Phase im Wesentlichen vollständig auflösen oder dispergiert werden können. Das wasserlösliche Polymermaterial wird aus der Gruppe der wasserlöslichen Polyurethane, der Polyethylenoxide, der Polyetheroxide, der Lignin und/oder Lignozellulose enthaltende Polymere oder deren Mischungen ausgewählt.

Eine Gruppe der bevorzugten Polymere sind die Lignin und/oder Lignozellulose enthaltenden Polymere, die sich sehr gut mittels Spritzguss verarbeiten lassen und zudem meist wasserlöslich oder wasserdispergierbar sind.

Als Lignin und/oder Lignozellulose enthaltende Polymere werden vorzugsweise Polymere eingesetzt, die neben Lignozellulose oder Lignin auch (Bio)polymere aus der Gruppe der Polyhydroxialkonoate, Polycarpolacton und Polyester sowie ggf. Harze aus der Gruppe der natürlichen Fettsäuren, der Wachse, der aliphatischen und aromatischen Ketone, Alkohole, Carbonsäuren, Lactone und Polycyclen in monomerer, oligomerer und polymerer Form enthalten. Solche Polymere lösen sich nach und nach in Wasser oder sind im Wasser dispergierbar - gegebenenfalls in Kombination mit Hilfsstoffen - und sind häufig biologisch abbaubar. Erhältlich sind solche Lignozellulose und/oder Lignin enthaltenden Polymere beispielsweise von der Firma Tecnaro GmbH, Ilsfeld Deutschland unter den Handelsnamen Arboblend® oder Arboform®.

Weiterhin kann der Werbeträger Mittel umfassen, die dem Polymermaterial eine Farbe verleihen, wie Farbstoffe oder Pigmente oder andere Stoffe mit einer Eigenfarbe. Diese dienen dazu, den Werbeträger einzufärben, so dass dieser eingebettet durch die transparente Phase hindurch gut erkannt werden kann und sich vorzugsweise auch gegenüber dem Hintergrund wie Reinigungsmittelformkörper, WC-Körbchen, Sanitärkeramik abhebt. Unter Farbe wird auch die Nichtfarbe weiß verstanden, so dass auch die Eigenfarbe weiß des Polymermaterials einen farbigen Werbeträger darstellen kann. In diesem Falle sind die Farbe verleihenden Mittel das Polymermaterial selbst. Sofern der Werbeträger selber transparent oder opak ist, wird die entsprechende andere Phase eingefärbt, um einen guten farblichen Kontrast zu erreichen.

Der Masse des Werbeträgers können weitere Additive wie Plastifiziermittel, Schmiermittel, Entformungsmittel oder Duftstoffe zugegeben werden. Auch Komponenten, die die Eigenschaften des Polymermaterials ändern, können zugesetzt werden.

Als Plastifiziermittel können bspw. Pentaerythritol wie Sorbitol, Mannitol, Glycerin und Glykole wie Glycerin, Ethylenglykol oder Polyethylenglykol zugesetzt werden. Feststoffe wie Talg, Stearinsäure, Alkali- und Erdalkalistearat, Siliziumdioxid, Zinkstearate, Glas-Mikro-Hohlkugeln oder kolloidales Kieselgel können ebenfalls eingesetzt werden.

Auch können der Masse des Werbeträgers ein oder mehrere Feststoffe zur Beschleunigung der Auflösegeschwindigkeit zugesetzt werden, wie bspw. Alkali- oder Erdalkalisalze wie Natrium-, Kalium-, Magnesium- oder Calciumhydrogencarbonat oder Carbonat in Verbindung mit einer Säure. Geeignete Säuren sind bspw. Säuren mit einer Carboxyl- oder Sulfonsäuregruppe oder deren Salze.

Sofern die Auflösegeschwindigkeit des Werbeträgers verlangsamt werden soll, können auch Hydrophobiermittel zugegeben werden, auch, um ein Quellen des Polymermaterials zu verhindern. Beispiele hierzu sind hydrophob modifizierte Aerosile vom Typ TS (TS 720) der Firma Degussa.

Auch anionische, nichtionische, kationische, amphotere oder zwitterionische oberflächenaktive Substanzen und deren Mischungen können der Werbeträgermasse zugesetzt werden. Durch Tenside kann auch die Löslichkeit des Werbeträgers beeinflusst und auch die Benetzbarkeit und die Schaumbildung des Systems beim Auflösen mit gesteuert werden.

Auch Schauminhibitoren oder Schaumbooster können dem Polymermaterial zugesetzt werden.

Eine weitere Klasse von Additiven, die dem Werbeträgermaterial zugesetzt werden können, sind Polymere. Hierunter fallen zum einen Polymere, die beim Reinigen Cobuildner-Eigenschaften zeigen, wie bspw. Polyacrylsäure, auch modifizierte Polyacrylsäuren oder entsprechende Copolymere.

Prinzipiell lassen sich auch Phosphate und Phosphonate als Additive dem Material des Werbeträgers zusetzen, um der Bildung von Urinstein und Kalkbelägen entgegen zu wirken.

Mit einem Zusatz von Nanopartikeln zu dem Polymer, wie beispielsweise kolloidalem SiO₂ (z.B. Ludox HS oder Ludox HM von Grace Davison) können easy-to-clean Effekte aufgebaut werden.

Weiterhin besteht die Möglichkeit, den Werbeträger mit kolloidalem Silber auszurüsten, um einen antibakteriellen Effekt zu erreichen. Geeignete Masterbatches zur Einarbeitung werden von der Firma FinePolymers zur Verfügung gestellt.

Der Werbeträger umfasst zwischen 1 und 99 Gew.%, vorzugsweise zwischen 5 und 90 Gew.% und besonders bevorzugt zwischen 10 und 70 Gew.% wasserlösliche oder wasserdispergierbare Polymermaterialien.

Additive können der Masse des Werbeträgers in einer Menge von bis zu 90 Gew.%, vorzugsweise in einer Menge von bis zu 70, Gew.% und besonders bevorzugt in einer Menge von 2 bis 30 Gew.%, zugesetzt werden.

Der Prozentsatz der der Masse des Werbeträgers hinzugefügten weiteren Bestandteile hängt u.a. auch von der Art des ausgewählten Polymermaterials ab. Wird ein Lignin und/oder Lignozellulose enthaltendes Polymermaterial ausgewählt, so sind schon Mengen von 7 bis 15 Gew.% des Polymermaterials in der Gesamtmasse ausreichend, d.h. der Anteil an Zusätzen, insbesondere von Tensid kann bis zu 80 oder 85 Gew.% betragen und von Füllstoffen bis zu 50 % bezogen auf die Tensidmenge.

Wird hingegen PVA als Polymermaterial ausgewählt, so sind schon Mengen von mindestens 20 Gew.% des Polymermaterials in der Gesamtmasse ausreichend, das heißt, der Anteil an Zusätzen, insbesondere von Tensid, kann bis zu 80 Gew.% betragen und der an Füllstoffen bis zu 50 %, bezogen auf die Tensidmenge.

Duftstoffe kann die Masse des Werbeträgers im Allgemeinen bis zu 10 Gew.% umfassen. Die Zugabe erfolgt direkt als Flüssigkeit oder über adsorbierte Parfüme auf großporigen Oberflächen, beispielsweise auf Na₂SO₄ oder auch in Form von gekapselten Materialien.

Prinzipiell ist es auch möglich, als Werbeträger beispielsweise PVA-Folien, die einen Flüssigkörper, eine Flüssigkeit oder ein Pulver umgeben, einzusetzen.

Die Herstellung des Werbeträgers erfolgt durch Spritzgießen. Beim Spritzgießen wird eine Formmasse derart umgeformt, dass die in einem Massezylinder für mehr als einen Spritzgießvorgang enthaltene Masse unter Wärmeeinwirkung plastisch erweicht und unter Druck durch eine Düse in den Hohlraum eines zuvor geschlossenen Werkzeugs einfließt. Dieses Verfahren wird hauptsächlich bei nicht härtbaren Formmassen angewandt, die im Werkzeug durch Abkühlung erstarren. Im praktischen Betrieb erwärmt man die thermoplastische Formmasse (Pulverkörner, Würfelpasten oder ähnliches) bis zur Verflüssigung und spritzt sie dann unter Druck in geschlossene zweiteilige, d.h. aus Matrize und Patrize bestehende, vorzugsweise wassergekühlte, Hohlformen ein, wo sie abkühlen und erstarren. Als Formmassen (Spritzgussmassen) eignen sich unter anderem die bereits beschriebenen wasserlöslichen Polymere umfassende Massen.

Als transparente Phase können beispielsweise transparente Gele oder transparente Seifenformulierungen eingesetzt werden, aber auch transparente wasserlösliche Kunststoffe oder transparente Folien. Seifenformulierungen sind beispielsweise aus der US 2001/004394 A1 bekannt und können neben Alkalicarbonsäuresalzen, Lösemitteln und Tensiden auch eine Zuckerverbindung umfassen.

Transparente Gele sind beispielsweise aus der EP 1 418 225 A1 oder der EP 1 553 162 A1 bekannt. Wesentliche Bestandteile der Gelphase sind Gelbildner und die für die Gelbildung erforderlichen Lösungsmittel.

Als Gelbildner können beispielsweise Metallseifen der höheren Fettsäuren, insbesondere die entsprechenden Alkalisalze wie Natriumstearat oder Natriumoleat, eingesetzt werden. Ebenfalls ist möglich, als Gelbildner nichtionische Tenside wie beispielsweise Polyalkoxyalkane, zum Beispiel ein Gemisch aus Alkyl-(C20,C22)-ethoxylat mit 35 EO, Alkyl-(C22)-ethoxylat mit 35 EO oder Alkyl-(C16,C18)-ethoxylat mit 30 EO, natürliche Polymere ("gums") wie Gummi arabicum, Guar-Gum, Agar Agar, Pektine, Gelatine, Stärke, Sorbitol, chemisch modifizierte oder derivatisierte natürliche "gums" wie Carboxymethylcellulose, Methylcellulose, Hydroxypropylmethylcellulose, Xanthan, Sorbitolderivate wie Dibenzylidensorbitol und deren Derivate, Stärkederivate wie Carboxymethylstärke, Hydroxyethylstärke, mikrobiell fermentierte "gums" wie Dextran, Polysaccharid B-1459 und Gelbildner wie Methoxypectin, Propylenglycolalginat, Triethanolaminalginat, Carboxymethyl-Guar-Gum etc. einzusetzen. Ebenfalls ist der Einsatz von Polyamiden oder anderen weniger hydrophilen Gelbildnern möglich.

Ein weiterer wesentlicher Bestandteil der Gelphase ist das Lösungsmittel. Prinzipiell kann ein organisches Lösungsmittel, Wasser oder deren Gemisch als Lösungsmittel eingesetzt werden. Neben Wasser sind insbesondere Alkylenglycolalkylether wie beispielsweise Propylenglycol-n-Buthylether, Dipropylenglycol-Methylether oder deren Gemisch als Lösungsmittel geeignet. Auch können als Lösungsmittel C₃ - C₅ Glykole, wie insbesondere Propylenglykol (zum Beispiel 1,2-Propandiol), Propylencarbonate, C₂ - C₄ Alkohole oder Polyalkylenglycole, vorzugsweise mit 200 - 600 Alkylenglycoleinheiten, Polyalkohole, (2)-Methyl-1,3-Propandiol, auch in Mischungen anderer Lösungsmittel, verwendet werden. Ein weiteres bevorzugtes Lösungsmittel sind die als Emulgator bekannten Fettalkoholethoxylate und deren Derivate wie zum Beispiel Hydroxyfettalkoholalkoxylat, wobei der Alkoxylatrest ein Copolymer aus Ethylen- und Propylenoxideinheiten ist und der Alkylrest aus einem C12/C16-Blend, vorzugsweise deren 2-Hydroxyverbindung, besteht. Diese Gruppe der bevorzugten Lösungsmittel sind bei der Firma Cognis unter der Marke "Eumulgin", zum Beispiel Eumulgin L, erhältlich.

Das beziehungsweise die Lösungsmittel sind im Allgemeinen polare Lösungsmittel, die die Gelbildner lösen. Ihr prozentualer Anteil beträgt zwischen 5 und 70 Gew.%.

Ist ein Teil des Lösungsmittels gleichzeitig ein Emulgator, so kann der Lösungsmittelanteil auf über 70 Gew.% steigen, zum Beispiel bis auf 80 Gew.%.

Weiterhin ist der Einsatz von weniger polaren Lösemitteln möglich, beispielsweise von Estern oder Kohlenwasserstoffen. Dabei werden die Polaritäten der Lösemittel immer auf den Gelbildner abgestimmt, damit dieser optimal vernetzen und quellen kann.

Weiterhin kann die Gelphase die zur permanenten Raumbeduftung erforderlichen Duftstoffe, insbesondere Parfümöle oder feste Riechstoffe wie Campher, enthalten. Die eingesetzten Parfümöle sind vorzugsweise hydrophob. Der Parfümanteil in der Gelphase hängt von der gewünschten Beduftung ab. Im Allgemeinen beträgt der Panfümanteil zwischen 2 und 70 Gew.%, vorzugsweise zwischen 5 und 20 Gew.% und besonders bevorzugt 8 bis 12 Gew.%.

Unter Parfum werden sämtliche duftende Rohstoffe des Mittels verstanden, auch wenn sie gleichzeitig Lösungsmittel, Lösungsretardierer etc. sind.

Die Gelphase kann zusätzlich einen oder mehrere Schäumer, wie beispielsweise Betaine, alkoxylierte Alkylethersulfate oder Lactobionsäurederivate oder deren Mischungen enthalten, wie beispielsweise Fettsäureamidopropyl-Betain mit einem C5 - C21-Fettsäureanteil wie beispielsweise Kokosamidoproplybetain, Alkali- oder Ammoniumsalze der Laurylethersulfate mit 1 bis 5 EO, Lactobionococylamid, Lactobionooleylamid, Lactobionotalgamid etc. oder deren Mischungen. Diese Schäumer lassen sich außerordentlich gut in die Gelphase einbringen. Besonders bevorzugt ist der Einsatz der flüssigen Superschäumer, nämlich der Betaine und der Laurylethersulfate.

Falls gewünscht, kann die Gelphase weiterhin Di-, Oligo- oder Polyhydroxyverbindungen oder deren Ether wie Glykol, 1,2- oder 1,3-Dihydroxypropan, 1,2-, 1,3-, 2,3- oder 1,4-Dihydroxybutan, die Isomere des Dihydroxyisobutans, Dihydroxypentans etc., Glycerin, Pentaerythrit, Penta- oder Hexahydroxyverbindungen oder Polyhydroxyether wie Polymethylen- oder Polypropylenglykol in einem Anteil von bis zu 20 Gew.% umfassen, um die Bildung schwerlöslicher Rückstände durch Austrocknung des Gels zu verhindern, den Auflösevorgang zu beschleunigen und eine ansehnlich glatte Oberfläche der Gelphase zu erreichen.

Zur Steuerung der Auflösegeschwindigkeit können der Gelphase zusätzlich Hydrophobiermittel wie beispielsweise Kokosfettsäuremonoethanolamid oder andere bekannte Hydrophobiermittel zugegeben werden; bevorzugt ist, zwischen 0 bis 10 Gew.% Hydrophobiermittel einzusetzen. Prinzipiell tragen auch die in der Gelphase enthaltenen hydrophoben Parfümöle zu einer Verminderung der Auflösegeschwindigkeit bei.

Soll die Auflösegeschwindigkeit hingegen erhöht werden, so setzt man Hydrotropika ein. Geeignete Hydrotropika sind Natriumtoluolsulfonat, Natriumcumolsulfonat, Natriumxylolsulfonat oder auch Natrium-Butylmonoglykolsulfat. Anstelle der Natriumsalze können auch andere geeignete Salze, insbesondere Alkalisalze, eingesetzt werden.

Der gewichtsprozentuale Anteil der Hydrotropika hängt von der gewünschten Absenkung der Lebensdauer der Gelphase ab. Häufig liegt er zwischen 0 und 10 Gew.%.

Vorzugsweise sollte der Schmelzpunkt der Gelphase - um eine formstabile Lagerung und Transport des Mittels zu gewährleisten - wenigstens 40° C, bevorzugt wenigstens 50° C, betragen.

Weiterhin kann als transparente Phase eine Seife eingesetzt werden, die wenigstens ein Salz einer Alkancarbonsäure, Lösemittel und Tenside umfasst.

Die transparente Phase umfasst wenigstens ein Alkancarbonsäuresalz, bevorzugt sind wenigstens zwei verschiedene und besonders bevorzugt drei verschiedene Alkancarbonsäuresalze. Eine besonders hohe Transparenz des Mittels konnte mit einem Gemisch aus Stearin-, Laurin- und Myristinsäuresalzen erzielt werden.

Die Gesamtmenge an Alkancarbonsäuren in der Alkancarbonsäuren und Alkalien umfassenden Ausgangsformulierung hängt von den Anwendungseigenschaften ab und liegt in der Regel zwischen 5 Gew.% und 30 Gew.%, vorzugsweise zwischen 10 Gew.% und 20 Gew.% und besonders bevorzugt zwischen 12 Gew.% und 18 Gew.%.

Die transparente Phase umfasst weitere Tenside, wobei die Tenside aus der Gruppe der anionischen, nichtionischen, kationischen oder amphoteren Tenside ausgewählt werden können oder Mischungen davon.

Unter den anionischen Tensiden sind insbesondere die Alkylsulfate, Alkylethersulfate, die Sulfonate wie beispielsweise die Alkylsulfonate, die Olefinsulfonate, die Alkoxyalkansulfonate, die Alkylarylsulfonate, die Sulfatester, die Alkylcarbonate, Alkylethercarboxylate, die Fettsäuretauride, die Alkylisethionate und deren Mischung bevorzugt.

Als nichtionische Tenside können beispielsweise Alkylethoxylate, ethoxylierte Alkylphenole, ethoxylierte oder propoxylierte Fettalkohole, Zuckertenside wie Alkylpolyglycoside, Polyethylenglykolether, ethoxylierte Fettsäureester, Kondensationsprodukte von Ethylenoxid mit langkettigen Aminen oder Amiden oder vergleichbaren Verbindungen, Aminoxiden, Trisiloxanalkoxylate oder deren Mischung eingesetzt werden.

Als amphotere Tenside können beispielsweise Betaine eingesetzt werden und als kationische Tenside quartäre Alkylammoniumverbindungen.

Im Falle des Einsatzes von kationischen Tensiden muss darauf geachtet werden, diese so auszuwählen, dass keine Wolkenbildung durch Bildung von unlöslichen Neutralkomplexen erfolgt.

Der Anteil an Tensiden richtet sich nach der gewünschten Reinigungs-/Schaumleistung und beträgt vorzugsweise zwischen 5 Gew.% und 25 Gew.% und insbesondere zwischen 8 Gew.% und 18 Gew.%.

Als Lösemittel können sowohl organische als auch anorganische Lösemittel eingesetzt werden. Bevorzugte Lösemittel sind Wasser und Alkohole, insbesondere mehrwertige Alkohole. Als Alkohole können beispielsweise 1,2-Propylenglykol, Dipropylenglykol, Butylenglykol, Ethylenglykol, 1,7-Heptandiol, Glycerin, Glycerinderivate, die Monoethylenglykole, die Polyethylenglykole mit einem Molekulargewicht bis zu 8000 sowie die Mono-C₁₋₄-Alkylether der vorangegangenen Verbindungen ausgewählt werden.

Um die gewünschte Transparenz zu erreichen, umfassen die Formulierungen auch Zucker oder Zuckerderivate, insbesondere Polyhydroxyaldosen oder -ketosen sowie deren niedere Oligomere, wie beispielsweise die Hexosen, Pentosen oder deren Di- oder Trisaccharide. Besonders bevorzugt sind unter den Monosacchariden die Glucose, Fructose, Arabiose, Ribose, Xylose, Mannose und Galactose und unter den Disacchariden die Saccharose, Maltose, Lactose, Raffinose und Cellobiose aus natürlichen oder synthetischen Quellen, unabhängig von ihrer Fischer-Konfiguration sowie alle Epimere, die auf diesen Zuckern basieren.

Zuckerderivate im Rahmen der vorliegenden Erfindung umfassen veretherte oder veresterte Zucker oder deren glycosidische (Acetal)-Form und substituierte Zucker oder deren Derivate wie Aminozucker und Thiozucker.

Als reduzierte oder oxidierte Formen der Zucker oder der Zuckerderivate können sämtliche unter Erhalt der C-Kette oxidierte oder reduzierte Zucker eingesetzt werden wie die Desoxyzucker, die Zuckeralkohole und die Zuckersäuren.

Der Anteil an reduzierten oder oxidierten Zuckern kann in dem Mittel bis zu 30 Gew.%, vorzugsweise bis zu 20 Gew.%, betragen und vorzugsweise wenigstens 15 Gew.%.

Eine besonders hohe Transparenz konnte bei einem Verhältnis von Zucker zu Zuckeralkohol von etwa 1:2 erzielt werden.

Als Salze der Alkancarbonsäuren werden im Rahmen der vorliegenden Erfindung die Salze der aliphatischen gesättigten oder ungesättigten Carbonsäuren eingesetzt. Vorzugsweise weisen die Alkancarbonsäuresalze eine Kohlenstoffkette mit zwischen 10 und 24 Kohlenstoffatomen auf und sind Monocarbonsäuresalze. Besonders bevorzugte Alkancarbonsäuresalze sind Salze von Laurin-, Myristin-, Palmitin-, Stearin-, Ölsäure und/oder C-Schnitte derselben aus natürlichen oder synthetischen Quellen.

Der transparenten Phase können weiterhin Parfümöle zugegeben werden, wobei Mengen bis zu 15 Gew.% und vorzugsweise zwischen 3 und 10 Gew.% und insbesondere etwa 5 Gew.% bevorzugt sind.

Der Erstarrungspunkt der transparenten Phase liegt in Abhängigkeit vom Wasser- und Fettsäureanteil zwischen 45 °C bis 70 °C und kann damit für die unterschiedlichsten Anwendungs- und Temperaturbereiche eingestellt werden.

Weiterhin kann eine nicht transparente Reinigungsmittelformkörperphase vorgesehen sein.

Unter Reinigungsmittelformkörper wird ein fester Körper, der vorzugsweise durch Extrusion oder Verpressen entsprechend der üblichen Herstellung von Rimblocks hergestellt ist, verstanden.

Der Reinigungsmittelformkörper umfasst Tenside, um die gewünschte Reinigungswirkung zu erzielen, nämlich kationische und/oder anionische und/oder nichtionische und/oder amphotere Tenside. Diese Tenside sollten zur Erzielung der gewünschten Reinigung gut netzende Tenside, somit vorzugsweise anionische oder nichtionische Tenside, sein. Prinzipiell sind alle bekannten anionischen Tenside geeignet. Vorzugsweise werden als anionische Tenside Alkylbenzolsulfonate, Alkylsulfate, Fettalkoholsulfate und Fettalkoholethersulfate eingesetzt. Vorzugsweise umfasst die Alkylgruppe oder der Fettsäurebestandteil zwischen 8 und 18 Kohlenstoffatome. Als Alkylbenzolsulfonat kann beispielsweise Natrium-alkyl-(C10-C13)-benzolsulfonat eingesetzt werden.

Weiterhin kann die zweite Phase Salze zur Regulierung der Konsistenz und des Abspülverhaltens enthalten. Im Allgemeinen handelt es sich bei diesen Salzen um anorganische Salze, die vorzugsweise aus der Gruppe der Alkali- und Erdalkalisalze der Schwefelsäuren, Phosphorsäuren, Stickstoffsäuren, Kohlensäure, Halogensäuren wie HCl oder deren Mischungen ausgewählt werden. Der Anteil an diesen Salzen in der zweiten Phase kann bis zu 80 Gew.% betragen, vorzugsweise etwa 20 bis 50 Gew.%. Besonders bevorzugt ist, als Salze Natriumchlorid oder Natriumsulfat oder deren Mischung einzusetzen.

Die zweite Phase kann weiterhin Extrusionshilfsmittel, vorzugsweise bis zu einem Anteil von 15 Gew.%, umfassen. Als Extrusionshilfsmittel können beispielsweise Alkylpolyethylenglycolether mit bis zu 40 EO, aber auch andere bekannte Extrusionshilfsmittel wie zum Beispiel Cellulose und ihre Derivate verwendet werden.

Weiterhin kann die zweite Phase Farbstoffe und Pigment wie beispielsweise Titandioxid umfassen.

Wahlweise kann die zweite Phase auch amphotere Tenside enthalten und Duftstoffe umfassen sowie auch Desinfektionsmittel, Konservierungsmittel, Bleichmittel, Aktivatoren, Enzyme, Komplexierungsmittel und Säuren umfassen.

Nachdem der farbige Spritzgusskörper eine Individualisierung des Produktes und eine ansprechende Optik bereitstellt, ist es nicht mehr erforderlich, zusätzlich auch noch das WC-Körbchen mit individualisierenden Motiven auf der Sichtseite auszugestalten, was die Produktion der Körbchen und die Materialauswahl - ausreichend ist jetzt ein einfaches transparentes Kunststoffkörbchen - vereinfacht und verbilligt.

Das erfindungsgemäße Reinigungsmittel mit Werbeträger kann beispielsweise durch folgende kontinuierliche und diskontinuierliche Verfahren herstellt werden:
a) Extrusion eines Reinigungsmittelformkörperstrangs (mit Aussparung), Einsetzen des Spritzgussteils in Linie, Übergießen mit Gel oder Seifenmasse, Abkühlung; Schneiden;
b) Kombinierte Extrusion des Reinigungsmittelformkörpers mit gleichzeitiger Befüllung mit Gel oder Seifenmasse und nachgeschalteter Einspeisung des Werbeträgers in die noch flüssige transparente Masse; Abkühlung; Schneiden;
c) Zwei transparente Massen von Rollmaterial werden zusammengeführt, wobei zwischen den beiden Massen der Werbeträger eingespeist wird; Schneiden;
d) Gießen der Seifenmasse, Einsetzen des Werbeträgers in die Masse, abkühlen lassen.
e) Überspritzen des Werbeträgers mit einer spritzbaren transparenten Masse
f) Ein Trennmittel, das gleichzeitig die transparente Phase bildet, wird in die Form eingespritzt und anschließend wird das Kunststoffgranulat zur Herstellung des Formkörpers in die Form eingeschossen. Hierdurch wird ein Formkörper erhalten, der an seiner Oberfläche eine dünne Schicht von transparentem Trennmittel aufweist.

Die Erfindung wird nachfolgend anhand von Ausführungsbeispielen näher beschrieben.

Verschiedene prinzipielle Ausgestaltungen des Mittels mit Werbeträger sind in den Figuren 1 bis 5 dargestellt.

Es zeigen
- Figur 1:: ein Mittel mit einem Werbeträger, der auf einer Seite be- reichsweise eine transparente Phase aufweist,
- Figur 2:: ein Mittel mit einem Werbeträger, der auf einer Seite voll- ständig eine transparente Phase aufweist,
- Figur 3:: ein Mittel mit einem Werbeträger, der auf drei Seiten von einer transparenten Phase umschlossen ist,
- Figur 4:: ein Mittel mit einem Werbeträger, der vollständig von ei- ner transparenten Phase umschlossen ist und
- Figur 5:: eine Draufsicht auf die Sichtfläche eines Werbeträgers.

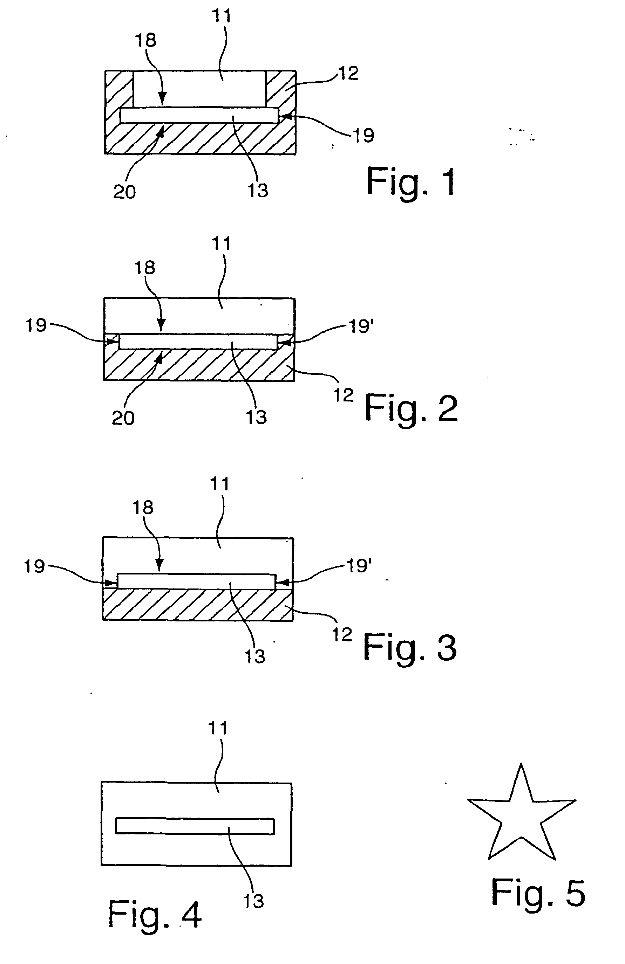

Das in **Fig. 1** dargestellte Mittel umfasst einen Werbeträger 13 auf, beispielsweise in Form eines Quaders, der auf der Sichtseite 18 bereichsweise mit einer transparenten Phase 11 beschichtet ist. Die der Sichtseite 18 gegenüberliegende Seite 20 und die beiden Seitenflächen 19 sind vollständig in eine Reinigungsmittelformkörpermasse 12 eingebettet, die beispielsweise extrudiert sein kann. Die Bereiche der Sichtseite 20 des Werbeträgers 13, die nicht mit der transparenten Phase 11 beschichtet sind, sind ebenfalls mit der Reinigungsmittelformkörperphase 12 beschichtet, so dass der Werbeträger vollständig eingebettet und gegen einen Angriff von Wasser geschützt ist.

Während des Gebrauchs werden die transparente Phase 11 und die Reinigungsmittelformkörperphase 12 erst nach und nach durch das Überströmen mit Spülwasser abgespült, und am Ende der Lebensdauer ist dann auch der Werbeträger 13 dem Angriff durch das Spülwasser ausgesetzt, durch den er sich dann ebenfalls vollständig auflöst.

**Figur 2** zeigt eine Variante, bei der der Werbeträger 13 in den Reinigungsmittelformkörper 12 eingebettet ist, so dass drei Seiten 19, 19' und 20 des Werbeträgers 13 mit dem Reinigungsmittelformkörper 12 bedeckt sind. Die Sichtseite 18 des Werbeträgers 13 ist über ihre gesamte Fläche mit der transparenten Phase 11 beschichtet.

Die Herstellung dieser Variante erfolgt derart, dass zunächst der Reinigungsmittelformkörper extrudiert und entsprechend geformt wird und in die in der Oberseite eingebrachte Ausnehmung dann der Werbeträger eingelegt und dieser anschließend mit der transparenten Phase beschichtet wird.

**Figur 3** zeigt eine Variante, bei der der Werbeträger 13 auf einer Seite an die Reinigungsmittelformkörperphase 12 angrenzt und auf drei Seiten 18, 19, 19' von der transparenten Phase 11 umgeben ist.

Eine Variante der Ausführungsformen gemäß den Figuren 2 oder 3 sieht vor, anstelle der Reinigungsmittelformkörperphase 12 eine zweite transparente Phase einzusetzen, so dass insgesamt ein in einem vollständig transparenten Mittel eingebetteter Werbeträger erhalten wird, wobei das transparente Mitte jedoch aus zwei unterschiedlichen transparenten Phasen besteht (beispielsweise eine transparente Gelphase und eine transparente Seifenphase).

In **Figur 4** ist der Werbeträger 13 vollständig von der transparenten Phase 11 umgeben.

**Figur 5** zeigt beispielhaft einen Werbeträger in Form eines Sterns.

Nachfolgend sind beispielhaft einige Rezepturen zur Herstellung des Werbeträgers, der transparenten Gelphase, der transparenten Seifenphase und der nichttransparenten Reinigungsmittelformkörperphase aufgeführt:

### I. Herstellung des Werbeträgers

### 1. Zusammensetzung der Spritzgussmasse zur Herstellung des Werbeträgers auf Basis eines ersten Polymers mit Lignin und Lignozellulose (Arboblend®):

Zur Herstellung des Probekörpers wurde Arboblend®-Granulat, erhältlich bei der Firma Tecnaro, Ilsfeld, eingesetzt.

Arboblend® ist ein zur Verarbeitung in Spritzmaschinen geeignetes Granulat, das aus den a) (Bio)Polymeren Polyhydroxialkanoat, Polycaprolacton, Polyester und Lignin, b) Lignozeltulose und c) Naturharzen, natürlichen Fettsäuren und Wachsen besteht.

In der nachfolgenden Tabelle ist die jeweilige Zusammensetzung des hergestellten Werbeträgers auf Arboblend®-Basis sowie das Verhalten beim Abspülen, die Oberflächenspannung, Schaumzahl und Auflösegeschwindigkeit aufgelistet:

**Tabelle 1**

| | *V1* | *V2* | *V3* | *V4* | *V5* |
|---|---|---|---|---|---|
| *Arboblend*® | *20%* | *15%* | *10%* | *10%* | *32%* |
| *Thermphos L500^{**}* | *0%* | *0%* | *0%* | *10%* | *0%* |
| *Marlon ARL ^{***}* | *80%* | *85%* | *90%* | *80%* | *68%* |
| | *100%* | *100%* | *100%* | *100%* | *100%* |
| *Einwaage [g]* | *8,58* | *9,08* | *8,84* | *9,07* | *9,42* |
| *Spülzahlen* | *339* | *206* | *112* | *198* | *265* |
| *Oberflächenspannung [mN*/*m] nach 100*/*500*/*1000 ms* | *66*/*48,5*/ *41,6* | *64,5*/*47,3*/ *40,8* | *63,6*/*45,8*/ *40* | *61*/*44,3*/ *38,8* | *64,4*/*46,8*/ *40,7* |
| *Schaumzahl [ml] nach 30 sek.*/*5 min.*/*30 min.* | *140*/*130*/ *125* | *140*/*140*/ *130* | *140*/*140*/ *130* | *140*/*130*/ *130* | *130*/*120*/ *120* |
| Auflösegeschwindigkeiten [Rührzeit in Stunden bei Raumtemperatur bei x % Rest] | 24h / 2% Rest | 24h / 5% Rest | 5h / gelöst | 5h / gelöst | 24h / 5% Rest |

| | | | | | |
|---|---|---|---|---|---|
| *(****)*Thermphos L500 ist Natriumtripolyphosphat und (***) Marlon ARL ist Natriumdodecylbenzolsulfonat. | | | | | |

Die mit den Polymeren auf Lignin- oder Lignozellulose-Basis hergestellten Formkörper weisen nur einen vergleichsweise geringen Anteil an Polymeren, nämlich zwischen 10 und 32 Gew.%, auf. Der Hauptbestandteil der Polymermasse sind Tenside.

Mit Arboblend lässt sich somit ein Polymer mit einem Füllgrad bis zu 90 % herstellen, das sich durch hohe Schaumzahlen, gute Netzfähigkeit und lange Standzeiten auszeichnet.

Bei einem Standard-Toilettenstein (Rim-Block) werden in der Regel Schaumzahlen von 100/90/90 ml nach 30 sek., 5 min. und 30 min., Oberflächenspannungen von 68/53/45 mN/m und Spülzahlen von 200 bis 250 erhalten.

Bei den mit Arboblend® hergestellten Formkörpern verbleiben nach dem Auflösen kleine Teilchen mit einer fadenartigen Struktur zurück, die auf die in dem Polymer enthaltenen Polyester zurückzuführen sind. Die kleinen Teilchen werden im Wesentlichen aus dem Körbchen herausgespült. Das Auflöseverhalten des Polyesters in dem Lignin-Polymer lässt sich weiterhin dadurch verbessern, dass anstelle des normalen Polyesters versprödetes Polyester bei der Herstellung des Polymeren auf Lingin/Lignozellulose-Basis eingesetzt wird.

Die Formkörper auf Lignin-/Lignozellulose-Basis lösen sich in Abhängigkeit von der jeweiligen Zusammensetzung etwa nach zwischen 100 und 350 Spülungen vollständig auf, und verbleibende kleine Rückstände werden aus dem Körbchen gespült, so dass die gewünschte Entleerung erreicht werden kann.

### 2. Zusammensetzung der Spritzgussmasse zur Herstellung des Werbeträgers auf Basis eines zweiten Polymers mit Lignin und Lignozellulose (Arboform®):

Zur Herstellung des Probekörpers wurde Arboform®-Granulat, erhältlich bei der Firma Tecnaro, Ilsfeld, eingesetzt. Arboform ist ein zur Verarbeitung in Spritzmaschinen geeignetes Granulat, das aus den a) Polymeren Lignin bzw. Ligninderivaten, biologisch abbaubaren Polyerstern, b) Lignozellulose bzw. Lignozellulotischen Fasern (Vergesellschaftung von Cellulose, Lignin und Holzpolyosen) und c) natürlichen Harzen, nämlich aliphatischen und aromatischen Ketonen, Alkoholen, Carbonsäuren, Lactonen und Polycyclen in monomerer, oligomerer und polymerer Form besteht.

In der nachfolgenden Tabelle ist die jeweilige Zusammensetzung des hergestellten Werbeträgers auf Arboform®-Basis sowie das Verhalten beim Abspülen, die Oberflächenspannung, Schaumzahl und Auflösegeschwindigkeit aufgelistet:

**Tabelle 2**

| | V1 | V2 | V3 |
|---|---|---|---|
| Arboform | 10 % | 15 % | 10 % |
| Tensid (Natriumdodecylbenzolsulfonat) | 90 % | 85 % | 88% |
| Farbstoff | - | - | 1 % |
| Duftstoff | - | - | 1 % |
| Einwaage [g] | 15,89 | 16,43 | 12,52 |
| Schaum [ml] nach 30 sek./5 min./30 min. | 120/115/115 | 135/125/125 | 130/120/100 |
| Netzzahl [mN/m] nach 100/500/1000 ms | 63,5/45,5/39,6 | 63/45/39 | 62/44,6/39 |
| Spülzahl | 76 | > 150 | > 150 |
| Auflösegeschwindigkeit [Rührzeit in Stunden bei Raumtemperatur bei x % Rest] | 30 min | 30 min | 25 min. |

Ergebnis: Die Werbeträger auf der Basis von Arboform® zeigen gute Performance in Bezug auf Schaum und Netzzahl, vergleichbare Standzeiten wie bei normalen Rimblocks, spülen weitestgehend linear ab und lassen sich gut formulieren. Ein weiterer Vorteil ist die Biokompatibilität ihrer Grundmasse.

### II. Ausführungsbeispiel einer transparenten Seifenmasse

Die Ausführungsbeispiele der transparenten Seifenmassen sind in der Anlage als Tabelle 6 aufgeführt.

### III. Ausführungsbeispiele für transparente Gele

### 1. Ausführungsbeispiel

**Tabelle 3**

| **Rohstoff** | **Rohstoffbezeichnung** | **Lieferant** | **Menge g** |
|---|---|---|---|
| Propylenglycol-n-Butylether | Propylenglycol-n-Butylether | DOW | 11 |
| Dipropylenglycol Monomethylether | Dipropylenglycol Monomethylether | DOW | 34 |
| Natriumstearat | Natriumstearat | Bärlocher | 8 |
| Kokosfettsäuremonoethanolamid | Comperlan 100 | Cognis | 5 |
| Natriumlauryl-Ethersulfat (2.5 EO) | Texapon N70 | Cognis | 19 |
| Parfum | Mint Fresh | Quest Gold- | 10 |
| Kokosamiodopropyl-Betain | Tego Betain F50 / Mackam 50-UL | schmidt/ Brenntag | 7.85 |
| Wasser | Wasser | | 9,15 |
| | | | 104 |

### 2. Ausführungsbeispiel

**Tabelle 4**

| **Rohstoff** | Rohstoffbezeichnung | Lieferant | Menge g |
|---|---|---|---|
| Wasser | | | 5 |
| 1,2 Propandiol | 1,2 Propandiol | Brenntag | 12,5 |
| PPG-1-PEG-9-Lauryl glycol ether | Eumulgin L | Cognis | 53 |
| Natriumstearat | Natriumstearat | Bärlocher | 8 |
| Na-Butylmonoglycolsulfat | Na-Butylmonoglycolsulfat | Hydrior | 4,5 |
| Parfum | Mint Fresh | Quest | 20 |
| | | | 103 |

### IV. Ausführungsbeispiel einer Reinigungsmittelformkörpermasse

**Tabelle 5**

| | | V1 | V2 |
|---|---|---|---|
| Rohstoff | Lieferant | Menge | Menge |
| Alkylbenzolsulfonat, Na-Salz | Unger | 66 | 17 |
| Natriumlaurylsulfat | Cognis | 0 | 30 |
| Natriumsulfat | Kemira | 20 | 20 |
| Luternsol AT 25 | BASF | 7 | 6 |
| Natriumcarbonat | Bilgram | 0 | 19 |
| Betaine | Goldschmidt | 1 | 2 |
| Farbe | BASF | <0,2 | <0,2 |
| Parfüm | Quest | 6 | 6 |
| Summe | | 100 | 100 |

### V. Experimentelles:

### 1. Bestimmung der Schaumzahlen:

### 1a) Herstellung der Stammlösung:

In einen 1 l Meßkolben werden 1,00 g Produkt eingewogen, mit demineralisiertem Wasser aufgelöst. Dazu werden 20 ml Calciumchlorid-Lösung gegeben, auf 20°C temperiert und mit demineralisiertem Wasser aufgefüllt.

Zur Herstellung der Calciumchlorid-Lösung werden 16,7 g Calciumchlorid Dihydrat p.a. in 21 demineralisiertem Wasser gelöst.

Die so hergestellte Stammlösung entspricht einer Wasserhärte von 6,4 °dH.

### 1b) Schaumzahl:

Man überführt 100 ml der auf 20 °C temperierten Stammlösung in einen 250 ml Mischzylinder und verschließt diese mit einem PTFE-Stopfen. Dann wird der Zylinder zwanzigmal hin und her gewendet (20 mal auf den Kopf gestellt). Nach jeweils: 30 sec/5 min/30 min wird das erzeugte Schaumvolumen (ml) abgelesen und notiert.

### 2. Bestimmung der Oberflächenspannung:

Gerät: Krüss BP 2 Blasendruckmeßgerät

### Prüfung Blindwert:

Mit dem Ufaryl DL 90 C wird eine 0,1%ige -Lösung hergestellt, bei 20°C dreimal eine Meßkurve mit dem vorher ermittelten Kapillardurchmesser aufgenommen und der Mittelwert gebildet. Die Abweichung vom Mittelwert darf maximal 1 mN/m sein.

Die Erstprüfung wird als Urwert genommen, alle Folgeprüfungen werden mit dem Urwert verglichen (50/100/500 ms). Bei Abweichung größer als ±2 mN/m ist die Kapillare durch eine neue auszutauschen.

Messung: Von einer 0,1%-igen Lösung (20 °C) wird die Oberflächenspannung in Abhängigkeit vom Alter der Oberfläche ermittelt. Betrachtet wird dabei die Oberflächenspannung nach 100, 500, 1000 ms.

### 3. Bestimmung der Auflösegeschwindigkeit:

Zur Bestimmung der Auflösegeschwindigkeit wurde der Formkörper bei Raumtemperatur in Wasser gegeben und gerührt, wie es bei der Bestimmung der Schaumzahlen beschrieben ist (1a). Allerdings wurde solange weitergerührt und die entsprechende Zeit bestimmt, bis sich der Formkörper vollständig aufgelöst oder dispergiert. Die Spülversuche wurden solange durchgeführt, bis eine weitestgehende Gewichtskonstanz des Materials eingetreten war.

### 4. Bestimmung der Spülzahl

Spülvolumen pro Spülung: 8 l

Spülrhythmus: 19 Spülungen am Tag nach einem definierten Schema.

Spülwassertemperatur: Die Spülwassertemperatur beträgt während der kurzen Spülfrequenzen (z.B. zwischen 3. und 5. Spülung) 13° - 14° C, während der langen Ruheperioden (z.B. zwischen 19. und 1. Spülung) 15° - 16°C. Die Temperatur des Spülwassers wird an jedem Werktag überprüft und notiert.

**Tabelle 6**

| **Rohstoff** | **Handelsbezeichnung** | **Lieferant** | v1 | v2 | v3 | v4 | v5 | v6 | v7 |
|---|---|---|---|---|---|---|---|---|---|
| 1,2 Propylenglycol | 1,2 Propylenglycol | Biesterfeld | 16,5 | 21,5 | 1,5 | 21,5 | 21,5 | 21,5 | 16 |
| Stearinsäure | Prifrac 2981 | Uniquema | 9,2 | 16 | 9,2 | 9,2 | 9,2 | 9,2 | 9,2 |
| Laurinsäure | Prifrac 2922 | Uniquema | 5 | 0 | 5 | 5 | 5 | 5 | 5 |
| Myristensäure | Prifrac 2942 | Uniquema | 1,8 | 0 | 1,8 | 1,8 | 1,8 | 1,8 | 1,8 |
| 1-Hydroxy-1,1-diphosphonsäure | Sequion 10 H 60 | Polygon | 0 | 0 | 0 | 0 | 0 | 0 | 0 |
| Wasser | ./. | | 9 | 9 | 9 | 9 | 9 | 9 | 9 |
| NaOH | Natriumhydroxid | Häffner | 2,5 | 2,5 | 2,5 | 2,5 | 0 | 2,5 | 2,5 |
| Ethylendiamintetraessigsäure-dinatriumsalz; | | | | | | | | | |
| flüssig | Trilon BD | BASF | 0 | 0 | 0 | 0 | 0 | 0 | 0 |
| Glycerin 86,5 % | Glycerin 86,5 % | Biesterfeld | 9 | 9 | 9 | 9 | 9 | 9 | 9 |
| C12-C14 Alkylethersulfat 1,3 EO | Texapon SP N 70 | Cognis | 10 | 10 | 10 | 10 | 10 | 0 | 0 |
| C12-C14 Alkylethersulfat 2,5 EO | Texapon N 70 | Cognis | | | | | 0 | 10 | 15,5 |
| Laurysulfat (94%) | Tensopol USP 94 | Manro | 3,5 | 3,5 | 3,5 | 3,5 | 3,5 | 3,5 | 3,5 |
| Sorbit, kristallin | | Suga | 17,8 | 17,8 | 17,8 | 17,8 | 17,8 | 17,8 | 17,8 |
| NaCl | ./. | | 0 | 0 | 0 | 0 | 0 | 0 | 0 |
| hydriertes Rizinusöl | Cremophor RLM 410 | BASF | 0 | 0 | 0 | 0 | 0 | 0 | 0 |
| Saccharose | Kristallzucker | Südzucker | 9 | 9 | 9 | 0 | 9 | 9 | 9 |
| Kalilauge, 50% | | Brenntag | 0,66 | 0,66 | 0,66 | 0,66 | 0 | 0,66 | 0,66 |
| Laurylethercarbonsäure | Akypo RLM 100 | Kao Chemicals | 0 | 0 | 0 | 0 | 0 | 0 | 0 |
| Cocoylisethionat | Hostapon KA | Clariant | | | | | 0 | | |
| TEA | Triethaniolamin, rein | BASF | | | | | | | |
| Parfüm | Orange Fun; # 561415 | Quest | 5 | | 20 | 0 | | | |
| Summe | | | **98,96** | **98,96** | **98,96** | **89,96** | **95,8** | **98,96** | **98,96** |

## Patentansprüche

1. Stückförmiges Reinigungsmittel für den Sanitärbereich, das wenigstens eine im Wesentlichen transparente Phase (11) und einen Werbeträger (13) aufweist,
wobei die transparente Phase (11) Reinigungsmittel umfasst und wasserlöslich oder wasserdispergierbar ist,
wobei der Werbeträger (13) ein durch thermische Verformung hergestelltes Formteil ist, welches wenigstens ein wasserlösliches oder wasserdispergierbares Polymermaterial aus der Gruppe der Lignin oder Lignacellulose enthaltenden Polymere, der wasserlöslichen Polyurethane, der Polyethylenoxide oder der Polyetheroxide enthält, wobei der Werbeträger (13) von der transparenten Phase (11) wenigstens teilweise umschlossen ist, der Werbeträger (13) wenigstens bereichsweise durch die transparente Phase (11) von außen sichtbar ist und der Werbeträger (13), dessen Dicke zwischen 3 mm und 50 mm, dessen Höhe zwischen 10 und 50 mm und dessen Breite zwischen 10 und 150 mm beträgt, in das Mittel eingebettet ist.

2. Stückförmiges Mittel nach Anspruch 1, **dadurch gekennzeichnet, dass** der Werbeträger (13) farbig ist oder sich farblich vom Hintergrund abhebt.

3. Stückförmiges Mittel nach einem der vorangegangenen Ansprüche , **dadurch gekennzeichnet, dass** der Werbeträger (13) vollständig in das Mittel eingebettet ist.

4. Stückförmiges Mittel gemäß Anspruch 3, **dadurch gekennzeichnet, dass** der Werbeträger (13) vollständig von der transparenten Phase (11) und/oder einer Reinigungsmittelphase (12) umschlossen ist.

5. Stückförmiges Mittel gemäß einem der Ansprüche 3 oder 4, **dadurch gekennzeichnet, dass** der Werbeträger (13) vollständig in der transparenten Phase (11) eingebettet ist.

6. Stückförmiges Mittel gemäß einem der vorangegangenen Ansprüche, **dadurch gekennzeichnet, dass** die transparente Phase (11) den Werbeträger (13) auf drei Seiten (18, 19, 19') kontaktiert.

7. Stückförmiges Mittel gemäß einem der vorangegangenen Ansprüche, **dadurch gekennzeichnet, dass** die Seiten oder Seitenbereiche des Werbeträgers (13), die nicht von der transparenten Phase (11) bedeckt sind, mit einer nichttransparenten Reinigungsmittelformkörperphase (12) bedeckt sind.

8. Stückförmiges Mittel gemäß einem der vorangegangenen Ansprüche, **dadurch gekennzeichnet, dass** die transparente Phase (11) ein transparentes Gel, eine transparente Seife, eine transparente Folie oder ein transparenter wasserlöslicher Kunststoff ist.

9. Stückförmiges Mittel gemäß einem der vorangegangenen Ansprüche, **dadurch gekennzeichnet, dass** die nichttransparente Reinigungsmittelformkörperphase ein extrudierter Reinigungsmittelformkörper ist.

10. Stückförmiges Mittel gemäß einem der vorangegangenen Ansprüche, **dadurch gekennzeichnet, dass** das Abspülverhalten im Wesentlichen linear ist.

11. Stückförmiges Mittel nach einem der vorangegangenen Ansprüche, **dadurch gekennzeichnet, dass** der Werbeträger anionische, kationische, nichtionische, zwitterionische Tenside umfasst.

12. Stückförmiges Mittel nach einem der vorangegangenen Ansprüche, **dadurch gekennzeichnet, dass** der Werbeträger Duftstoffe enthält.

13. Verfahren zur Herstellung eines stückförmigen Reinigungsmittels nach Anspruch 1, **dadurch gekennzeichnet, dass** der Werbeträger (13) durch thermisches Pressen, Blasverfahren, Spritzguss oder Tiefziehen hergestellt wird.

14. Verfahren zur Herstellung eines stückförmigen Mittels gemäß einem der vorangegangenen Ansprüche, **dadurch gekennzeichnet, dass** das Spritzgussteil spanend oder stanzend bearbeitet oder thermisch umgeformt wird.

## Claims

1. Bar-shaped cleaning composition for the sanitary sector, which has at least one essentially transparent phase (11) and an advertising medium (13),
where the transparent phase (11) comprises cleaning composition and is water-soluble or water-dispersible,
where the advertising medium (13) is a moulding produced by thermal shaping which comprises at least one water-soluble or water-dispersible polymer material from the group of polymers comprising lignin or lignocellulose, of water-soluble polyurethanes, of polyethylene oxides or of polyether oxides, where the advertising medium (13) is at least partially surrounded by the transparent phase (11), the advertising medium (13) is visible externally at least in areas through the transparent phase (11) and the advertising medium (13), the thickness of which is between 3 mm and 50 mm, the height of which is between 10 and 50 mm and the width of which is between 10 and 150 mm, is embedded into the composition.

2. Bar-shaped composition according to Claim 1, **characterized in that** the advertising medium (13) is coloured or stands out from the background in terms of colour.

3. Bar-shaped composition according to one of the preceding claims, **characterized in that** the advertising medium (13) is embedded completely into the composition.

4. Bar-shaped composition according to Claim 3, **characterized in that** the advertising medium (13) is surrounded completely by the transparent phase (11) and/or a cleaning composition phase (12).

5. Bar-shaped composition according to either of Claims 3 and 4, **characterized in that** the advertising medium (13) is embedded completely in the transparent phase (11).

6. Bar-shaped composition according to one of the preceding claims, **characterized in that** the transparent phase (11) makes contact with the advertising medium (13) on three sides (18, 19, 19').

7. Bar-shaped composition according to one of the preceding claims, **characterized in that** the sides or side areas of the advertising medium (13) which are not covered by the transparent phase (11) are covered with a non-transparent cleaning composition shaped-body phase (12).

8. Bar-shaped composition according to one of the preceding claims, **characterized in that** the transparent phase (11) is a transparent gel, a transparent soap, a transparent film or a transparent water-soluble plastic.

9. Bar-shaped composition according to one of the preceding claims, **characterized in that** the non-transparent cleaning composition shaped-body phase is an extruded cleaning composition shaped-body.

10. Bar-shaped composition according to one of the preceding claims, **characterized in that** the flush-away behaviour is essentially linear.

11. Bar-shaped composition according to one of the preceding claims, **characterized in that** the advertising medium comprises anionic, cationic, non-ionic, zwitterionic surfactants.

12. Bar-shaped composition according to one of the preceding claims, **characterized in that** the advertising medium comprises fragrances.

13. Method of producing a bar-shaped cleaning composition according to Claim 1, **characterized in that** the advertising medium (13) is produced by thermal compression, blow-moulding process, injection moulding or deep-drawing.

14. Method of producing a bar-shaped composition according to one of the preceding claims, **characterized in that** the injection moulding is machined or stamped or thermoformed.

## Revendications

1. Produit de nettoyage en bloc pour le domaine sanitaire, qui comporte au moins une phase essentiellement transparente (11) et un support publicitaire (13),
la phase transparente (11) comprenant un produit de nettoyage et étant soluble dans l'eau ou dispersable dans l'eau,
le support publicitaire (13) étant une pièce moulée produite par formage thermique, qui contient au moins une matière polymère soluble dans l'eau ou dispersable dans l'eau, choisie dans le groupe des polymères contenant de la lignine ou de la lignocellulose, des polyuréthannes hydrosolubles, des polyoxyéthylènes ou des polyétheroxydes, le support publicitaire (13) étant entouré au moins en partie par la phase transparente (11), le support publicitaire (13) étant au moins par zones visible de l'extérieur à travers la phase transparente (11) et le support publicitaire (13), dont l'épaisseur est comprise entre 3 mm et 50 mm, dont la hauteur est comprise entre 10 et 50 mm et dont la largeur est comprise entre 10 et 150 mm, étant encastré dans le produit.

2. Produit en bloc selon la revendication 1, **caractérisé en ce que** le support publicitaire (13) est coloré ou ressort sur le fond par la couleur.

3. Produit en bloc selon l'une quelconque des revendications précédentes, **caractérisé en ce que** le support publicitaire (13) est totalement encastré dans le produit.

4. Produit en bloc selon la revendication 3, **caractérisé en ce que** le support publicitaire (13) est complètement entouré par la phase transparente (11) et/ou par une phase de produit de nettoyage (12).

5. Produit en bloc selon l'une quelconque des revendications 3 ou 4, **caractérisé en ce que** le support publicitaire (13) est complètement encastré dans la phase transparente (11).

6. Produit en bloc selon l'une quelconque des revendications précédentes, **caractérisé en ce que** la phase transparente (11) est en contact sur trois côtés (18, 19, 19') avec le support publicitaire.

7. Produit en bloc selon l'une quelconque des revendications précédentes, **caractérisé en ce que** les côtés ou zones des côtés du support publicitaire (13), qui ne sont pas recouvert(e)s par la phase transparente (11), sont recouvert (e) s par une phase de corps moulé de produit de nettoyage (12) non transparente.

8. Produit en bloc selon l'une quelconque des revendications précédentes, **caractérisé en ce que** la phase transparente (11) est un gel transparent, un savon transparent, un film transparent ou une matière plastique hydrosoluble transparente.

9. Produit en bloc selon l'une quelconque des revendications précédentes, **caractérisé en ce que** la phase de corps moulé de produit de nettoyage non transparente est un corps moulé de produit de nettoyage extrudé.

10. Produit en bloc selon l'une quelconque des revendications précédentes, **caractérisé en ce que** le comportement d'érosion est essentiellement linéaire.

11. Produit en bloc selon l'une quelconque des revendications précédentes, **caractérisé en ce que** le support publicitaire comprend des tensioactifs anioniques, cationiques, non ioniques, zwitterioniques.

12. Produit en bloc selon l'une quelconque des revendications précédentes, **caractérisé en ce que** le support publicitaire contient des parfums.

13. Procédé pour la production d'un produit de nettoyage en bloc selon la revendication 1, **caractérisé en ce que** le support publicitaire (13) est produit par pressage thermique, un procédé de soufflage, moulage par injection ou emboutissage.

14. Procédé pour la production d'un produit en bloc selon l'une quelconque des revendications précédentes, **caractérisé en ce que** la pièce moulée par injection est façonnée par enlèvement de copeaux ou par poinçonnage ou mise en forme thermiquement.
